# EUROPEAN PATENT APPLICATION

(11) **EP 4 424 833 A2**
(43) Date of publication of application: **04.09.2024**
(21) Application number: 24189213.2
(22) Date of filing: 12.03.2014
(51) Int. Cl.: C12P 7/56

(54) **A SYSTEM AND METHOD FOR CONTROLLING METABOLITE PRODUCTION IN A MICROBIAL FERMENTATION**

(30) Priority: 15.03.2013 US 201361791065 P
(62) Divisional of application: 14768693.5
(71) Applicant: LanzaTech NZ, Inc., Wilmington, New Castle Delaware 19801 (US)
(72) Inventor: SIMPSON, Sean, Dennis, 1052 Parnell Auckland (NZ); KOEPKE, Michael, 1052 Parnell Auckland (NZ); SMART, Kathleen, Frances, 1052 Parnell Auckland (NZ); TRAN, Loan, Phuong, 1052 Parnell Auckland (NZ); SECHRIST, Paul, Roselle, 60172 (US)
(74) Representative: J A Kemp LLP

(57) **Abstract**

A method is provided for controlling a metabolic profile of an anaerobic microbial fermentation culture. In particular, a metabolic profile of a fermentation process is controlled by controlling the amount of dissolved CO2 provided to a culture. Further provided is a method of producing one or more products by microbial fermentation of a gaseous substrate through feeding tail gas CO2 from a reactor to a second reactor, or by recycling tail gas CO2 to the same reactor.

## Description

### FIELD OF THE INVENTION

This invention relates generally to methods for controlling the production of one or more products, by microbial fermentation. In particular, the invention relates to methods for controlling the amount of carbon dioxide provided to a microbial culture. In particular embodiments, a metabolic profile of a fermentation process is controlled by controlling the amount of dissolved CO2 provided to a culture.

### BACKGROUND OF THE INVENTION

Ethanol is rapidly becoming a major hydrogen-rich liquid transport fuel around the world. Worldwide consumption of ethanol in 2002 was an estimated 10.8 billion gallons. The global market for the fuel ethanol industry has also been predicted to grow sharply in future, due to an increased interest in ethanol in Europe, Japan, the USA and several developing nations.

For example, in the USA, ethanol is used to produce E10, a 10% mixture of ethanol in gasoline. In E10 blends the ethanol component acts as an oxygenating agent, improving the efficiency of combustion and reducing the production of air pollutants. In Brazil, ethanol satisfies approximately 30% of the transport fuel demand, as both an oxygenating agent blended in gasoline, or as a pure fuel in its own right. Also, in Europe, environmental concerns surrounding the consequences of Green House Gas (GHG) emissions have been the stimulus for the European Union (EU) to set member nations a mandated target for the consumption of sustainable transport fuels such as biomass derived ethanol.

The vast majority of fuel ethanol is produced via traditional yeast-based fermentation processes that use crop derived carbohydrates, such as sucrose extracted from sugarcane or starch extracted from grain crops, as the main carbon source. However, the cost of these carbohydrate feed stocks is influenced by their value as human food or animal feed, while the cultivation of starch or sucrose-producing crops for ethanol production is not economically sustainable in all geographies. Therefore, it is of interest to develop technologies to convert lower cost and/or more abundant carbon resources into fuel ethanol.

CO is a major free energy-rich by-product of the incomplete combustion of organic materials such as coal or oil and oil derived products. For example, the steel industry in Australia is reported to produce and release into the atmosphere over 500,000 tonnes of CO annually.

It has long been recognised that catalytic processes may be used to convert gases consisting primarily of CO and/or CO and hydrogen (H₂) into a variety of fuels and chemicals. However, micro-organisms may also be used to convert these gases into fuels and chemicals. These biological processes, although generally slower than chemical reactions, have several advantages over catalytic processes, including higher specificity, higher yields, lower energy costs and greater resistance to poisoning.

The ability of micro-organisms to grow on CO as their sole carbon source was first discovered in 1903. This was later determined to be a property of organisms that use the acetyl coenzyme A (acetyl CoA) biochemical pathway of autotrophic growth (also known as the Woods-Ljungdahl pathway and the carbon monoxide dehydrogenase / acetyl CoA synthase (CODH/ACS) pathway). A large number of anaerobic organisms including carboxydotrophic, photosynthetic, methanogenic and acetogenic organisms have been shown to metabolize CO to various end products, namely CO₂, H₂, methane, n-butanol, acetate and ethanol. While using CO as the sole carbon source all such organisms produce at least two of these end products.

Anaerobic bacteria, such as those from the genus *Clostridium,* have been demonstrated to produce ethanol from CO, CO₂ and H₂ via the acetyl CoA biochemical pathway. For example, various strains of *Clostridium ljungdahlii* that produce ethanol from gases are described in WO 00/68407, EP 117309, US patent nos. 5,173,429, 5,593,886, and 6,368,819, WO 98/00558 and WO 02/08438. The bacterium *Clostridium autoethanogenum sp* is also known to produce ethanol from gases (Abrini et al, Archives of Microbiology 161, pp 345-351 (1994)).

However, ethanol production by micro-organisms by fermentation of gases is always associated with co-production of acetate and/or acetic acid. As some of the available carbon is converted into acetate/acetic acid rather than ethanol, the efficiency of production of ethanol using such fermentation processes may be less than desirable. Also, unless the acetate/acetic acid by-product can be used for some other purpose, it may pose a waste disposal problem. Acetate/acetic acid is converted to methane by micro-organisms and therefore has the potential to contribute to Green House Gas emissions.

The importance of controlling parameters of the liquid nutrient medium used for culturing bacteria or micro-organisms within a bioreactor used for fermentation has been recognised in the art. NZ 556615, filed 18 July 2007 and incorporated herein by reference, describes, in particular, manipulation of the pH and the redox potential of such a liquid nutrient medium. For example, in the culture of anaerobic acetogenic bacteria, by elevating the pH of the culture to above about 5.7 while maintaining the redox potential of the culture at a low level (-400 mV or below), the bacteria convert acetate produced as a by-product of fermentation to ethanol at a much higher rate than under lower pH conditions. NZ 556615 further recognises that different pH levels and redox potentials may be used to optimise conditions depending on the primary role the bacteria are performing (i.e., growing, producing ethanol from acetate and a gaseous CO-containing substrate, or producing ethanol from a gaseous containing substrate).

US 7,078,201 and WO 02/08438 also describe improving fermentation processes for producing ethanol by varying conditions (e.g. pH and redox potential) of the liquid nutrient medium in which the fermentation is performed.

The pH of the liquid nutrient medium may be adjusted by adding one or more pH adjusting agents or buffers to the medium. For example, bases such as NaOH and acids such as sulphuric acid may be used to increase or decrease the pH as required. The redox potential may be adjusted by adding one or more reducing agents (e.g. methyl viologen) or oxidising agents. Alternatively the pH of the medium may be adjusted by providing an excess amount of the gaseous substrate to the fermentation such that the microorganisms are "oversupplied" with gas.

Similar processes may be used to produce other alcohols, such as butanol, as would be apparent to one of skill in the art.

It is an object of the present invention to provide a system and/or a process that goes at least some way towards overcoming the above disadvantages, or at least to provide the public with a useful choice.

### SUMMARY OF THE INVENTION

In a first aspect of the invention there is provided a method for controlling the metabolic profile of a fermentation culture comprising at least one carboxydotrophic acetogenic microorganism, the method comprising:
a. flowing a gaseous substrate comprising CO and CO2 to a bioreactor comprising a culture of the microorganism in a liquid nutrient medium; and
b. adjusting the amount of CO2 dissolved in the culture such that the metabolism of the culture is altered.

In one embodiment the amount of CO2 dissolved in the liquid nutrient medium is adjusted by controlling the flow of CO2 to the bioreactor. In one embodiment, increasing the amount of CO2 dissolved in the liquid nutrient medium alters the metabolism of the microorganism such that the production of one or more products derived from pyruvate is increased. In one embodiment, decreasing the amount of CO2 dissolved in the liquid nutrient medium alters the metabolism of the microorganism such that the production of one or more products derived from pyruvate is decreased.

In one embodiment the one or more products derived from pyruvate is selected from the group consisting of 2,3-butanediol (2,3-BDO), lactate, succinate, methyl ethyl ketone (MEK), 2-butanol, propanediol, 2-propanol, isopropanol, acetoin, iso-butanol, citramalate, butadiene, and poly lactic acid (PLA) .

In one embodiment, the fermentation is carried out at a pressure of about 250 to about 450 kPag (or greater than 500 kPag), such that the concentration of CO2 dissolved in the liquid nutrient medium is increased. In certain embodiments, the pressure is greater than 250 kPag or greater than 300 kPag, or greater than 350 kPag, or greater than 400 kPag, or greater than 450 kPag, or greater than 500 kPag.

In an alternative embodiment, the pressure in the reactor is reduced or minimised to promote the production of one or more products derived from acetyl coA compared to one or more products derived from pyruvate. In certain embodiments, the pressure in the bioreactor is from about atmospheric to about 200 kPag or is maintained below 200 kPag, or less than 150 kPag, or less than 100 kPag, or less than 50 kPag, or at atmospheric pressure.

In one embodiment the CO2 partial pressure is increased, to increase the amount of CO2 dissolved in the liquid nutrient medium.

In one embodiment, the amount of CO2 dissolved in the liquid nutrient medium is increased by increasing the amount of CO2 in the gaseous substrate provided to the fermentation. In one embodiment the concentration of CO2 in the substrate provided to the bioreactor is at least 10%, or at least 15%, or at least 18%, or at least 20%, or at least 25%, or at least 30%, or at least 35%, or at least 40%, or at least 45%. In certain embodiments, the concentration of CO2 in the substrate provided to the bioreactor is between 15% and 65%, or from about 20% to about 50%, or from about 25% to about 45%. In embodiments where pressure is applied to the fermentation, the amount of CO2 required by the fermentation is reduced. In the presence of pressure greater than about 50 kPag, the amount of CO2 provided in the substrate stream is substantially less than when provided at atmospheric pressure. In particular embodiments, the concentration of CO2 in the substrate provided to the bioreactor is from about 1% to about 50% when supplied at a pressure of greater than about 50 kPag.

In a second aspect of the invention there is provided a method for increasing the production of at least one product derived from pyruvate, the method comprising:
a. flowing a substrate comprising CO and CO2 to a bioreactor comprising a culture of at least one carboxydotrophic acetogenic microorganism in a liquid nutrient medium; and
b. adjusting the amount of CO2 flowed to the bioreactor such that the amount of dissolved CO2 provided in the liquid nutrient medium is increased.

In a third aspect of the invention, there is provided a method for controlling a ratio of pyruvate derived products to acetyl co-A derived products, the method comprising;
a. flowing a substrate comprising CO and CO2 to a bioreactor comprising a culture of at least one carboxydotrophic acetogenic microorganism in a liquid nutrient medium; and
b. adjusting the flow of carbon dioxide to the bioreactor such that the amount of CO2 dissolved in the liquid nutrient medium thereby controlling the ratio of pyruvate derived products to acetyl CoA derived products.

In one embodiment of the invention, increasing the amount of CO2 dissolved in the liquid nutrient medium increases the ratio of pyruvate derived products to acetyl CoA derived products by increasing the production of pyruvate derived products. In one embodiment, decreasing the amount of dissolved CO2 in the liquid nutrient medium decreases the ratio of pyruvate derived products to Acetyl CoA derived products by decreasing the production of pyruvate derived products.

In a fourth aspect there is provided a method for controlling the metabolic profile of a fermentation culture comprising at least one carboxydotrophic acetogenic microorganism, the method comprising
a. flowing a gaseous substrate comprising CO and CO2 to a bioreactor comprising a culture of the microorganism in a liquid nutrient medium;
b. monitoring the CO2 concentration in an exit stream exiting the bioreactor; and
c. adjusting the amount of CO2 dissolved in the liquid nutrient medium such that the metabolism of the culture is controlled.

In a fifth aspect there is provided a method for increasing the production of one or more products the method comprising;
a. providing a substrate comprising CO to a bioreactor containing a culture of one or more microorganisms in a liquid nutrient medium; and
b. fermenting the substrate to produce one or more liquid products and CO2.

In one embodiment one or more fermentation conditions are adjusted to increase the amount CO consumed by the culture and the amount of CO2 produced by the culture. In one embodiment the amount of CO consumed by the culture is increased by altering mass transfer in the fermentation. In one embodiment, the amount of CO consumed by the culture is increased by increasing the rate of flow of the gaseous substrate to the bioreactor. In one embodiment the amount of CO consumed by the culture is increased by increasing a rate of agitation of the liquid nutrient medium in the bioreactor. In one embodiment the amount of CO consumed by the culture is increased by increasing a bubble surface area.

In one embodiment, increasing the amount of CO consumed by the microbial culture increases the amount of CO2 in an outlet stream exiting the bioreactor. In one embodiment, the amount of CO2 in the outlet stream is at least 30%, or at least 35%, or at least 40%, or at least 45%, or at least 50%.

In a sixth aspect, there is provided a method for increasing the amount of dissolved CO2 in a liquid nutrient medium comprising a culture of at least one microorganism, the method comprising;
a. introducing a feed gas stream comprising CO and a liquid nutrient medium to at least one bioreactor to form a fermentation broth, the bioreactor further comprising a downcomer for circulating a portion of the fermentation broth from a point near the top of the bioreactor to a point near the bottom of the bioreactor;
b. fermenting the CO in the bioreactor to liquid products and a gas exit stream comprising CO2;
c. passing at least a portion of the gas exit stream to either the downcomer of the bioreactor which is the source of the gas exit stream located near the top of the bioreactor or to a second bioreactor; and
d. mixing the gas exit stream and the liquid nutrient medium along the downcomer to form a gas-liquid mixture thereby increasing the hydrostatic pressure above the gas-liquid mixture, such that CO2 from the exit gas stream is dissolved into the liquid nutrient medium at the bottom portion of the down comer.

In a specific embodiment the exit gas stream from the first bioreactor is passed to the downcomer of a second bioreactor. In another embodiment the exit gas stream from the first bioreactor is recycled to the downcomer of the first bioreactor. Alternatively, the exit gas stream from the first bioreactor is passed to the gas inlet of either the first or second bioreactor. Additionally, the feed stream to the second reactor can be a portion of the exit or tail gas stream from the first reactor optionally mixed with fresh feed gas stream. Additional bioreactors can be added in series and exit gas streams passed to the same or different bioreactors as described above.

In a further aspect there is provided a method for producing one or more products by microbial fermentation of a gaseous substrate, the method comprising:
a. In a first reactor comprising a culture of one or more carboxydotrophic microorganism in a liquid nutrient medium, receiving a gaseous substrate comprising CO;
b. fermenting the gaseous substrate comprising CO to produce one or more liquid products and an exit gas comprising CO2;
c. feeding the exit gas comprising CO2 to a second bioreactor, said second bioreactor comprises a culture of one or more carboxydotrophic microorganism in a liquid nutrient medium; and
d. fermenting the exit gas comprising CO2 to produce one or more products.

In one embodiment, the exit gas comprising CO2 is blended with one or more gaseous substrates prior to being fed to the second bioreactor. In one embodiment, an additional gaseous substrate is added to the second bioreactor for use as substrates in the microbial fermentation.

In one embodiment the one or more microorganism provided in the first bioreactor and the second bioreactor is the same. In one embodiment the microbial fermentation produces at least two products. In one embodiment the production ratio of the two products is different between the first bioreactor and the second bioreactor. In one embodiment, the fermentation produces at least one alcohol and at least one by-product. In one embodiment the ratio of the at least one product to the at least one by-product is different in the first and second bioreactors. In one embodiment the product is ethanol and the by-product is 2,3-butanediol (2,3-BDO). In one embodiment the ratio of ethanol (EtOH) to 2,3-BDO is lower in the second bioreactor.

In one embodiment the one or more microorganism is selected from the group comprising *Clostridium autoethanogenum, Clostridium ljundgahlii, Clostridium ragsdalei, Clostridium carboxydivorans,* and *Clostridium coskatii.*

In one embodiment a tail gas exiting the second bioreactor can be recycled to the first bioreactor for use as a substrate.

In a further aspect of the invention there is provided a method for controlling the metabolic profile of a fermentation culture comprising at least one carboxydotrophic acetogenic microorganism, the method comprising;
a. flowing a gaseous substrate comprising CO to a bioreactor comprising a culture of the microorganism in a liquid nutrient medium to provide a fermentation broth; and
b. increasing a rate of CO oxidation via a ferredoxin dependent carbon monoxide dehydrogenase to increase a level of reduced ferredoxin in the fermentation broth;
wherein the increased level of reduced ferredoxin increases a rate of pyruvate fermentation from acetyl coA.

### BRIEF DESCRIPTION OF THE DRAWINGS

Figure 1 shows the metabolic pathway of the micro-organisms of the present invention.
Figure 2 is a graph showing the effect of pressure on metabolite concentrations during fermentation.
Figure 3 is a graph showing the effect of dissolved CO2 in the liquid nutrient medium on 2,3-butanediol production.
Figure 4 is a graph showing the CO utilisation of the microbial culture of example 2.
Figure 5 is a graph showing the effect of CO2 concentration in the inlet stream on metabolite concentration for example 3A.
Figure 6 is a graph showing the uptake of CO, CO₂ and H₂ by the microbial culture for example 3A.
Figure 7 is a graph showing the concentration of metabolites over time for example 3B.
Figure 8 is a graph showing the gas composition for example 3B.
Figure 9 is a graph showing the uptake of various components of the inlet gas stream of example 3C by the microbial culture.
Figure 10 is a graph showing the effect of incrementally increasing the CO₂ in the inlet gas stream on metabolite concentration for example 3C.
Figure 11 is a graph showing metabolite concentrations where the concentration of CO2 in the inlet stream is cycled according to example 3D.
Figure 12 is a graph showing uptake of various components in the inlet stream of example 3D by the microbial culture.
Figure 13 is a graph showing metabolite concentrations for example 3E.
Figure 14 is a graph showing uptake of various components in the inlet stream of example 3E by the microbial culture.
Figure 15 is a graph showing the metabolite concentrations for example 4.
Figure 16 is a plot of calculated dissolved CO₂ versus 2,3 butanediol production rate.
Figure 17 is a representation of a system according to one embodiment of the invention.
Figure 18 is a graph showing the uptake of various components in the inlet stream of example 4 by the microbial culture.

### DETAILED DESCRIPTION

The inventors have discovered methods and systems for controlling the metabolic products produced by a culture of one or more carboxydotrophic acetogenic microorganism. In particular the inventors have found a method for increasing the production of one or more products derived from pyruvate in a fermentation process.

The following is a description of the present invention, including preferred embodiments thereof, given in general terms. The invention is further exemplified in the disclosure given under the heading "Examples" herein below, which provides experimental data supporting the invention, specific examples of aspects of the invention, and means of performing the invention.

### Definitions

As used herein "butanediol" refers to all structural isomers of the diol including 1,2-butanediol, 1,3-butanediol, 1,4-butanediol and 2,3-butanediol and stereoisomers thereof. The term "2,3-butanediol" should be interpreted to include all enantiomeric and diastereomeric forms of the compound, including (R,R), (S,S) and meso forms, in racemic, partially stereoisomerically pure and/or substantially stereoisomerically pure forms.

The term "bioreactor" includes a fermentation device consisting of one or more vessels and/or towers or piping arrangement, which includes the Continuous Stirred Tank Reactor (CSTR), Immobilized Cell Reactor (ICR), Trickle Bed Reactor (TBR), Bubble Column, Gas Lift Fermenter, Static Mixer, or other vessel or other device suitable for gas-liquid contact. As is described herein after, in some embodiments the bioreactor may comprise a first growth reactor and a second fermentation reactor. As such, when referring to the addition of a substrate, for example a substrate comprising carbon monoxide, to the bioreactor or fermentation reaction it should be understood to include addition to either or both of these reactors where appropriate.

The term "substrate comprising carbon monoxide" and like terms should be understood to include any substrate in which carbon monoxide is available to one or more strains of bacteria for growth and/or fermentation, for example.

"Gaseous substrates comprising carbon monoxide" include any gas which contains a level of carbon monoxide. The gaseous substrate will typically contain a major proportion of CO, preferably at least about 15% to about 95% CO by volume.

"Substrate comprising CO2" includes any substrate stream which contains a level of carbon dioxide. However, it should be appreciated that the gaseous substrate may be provided in alternative forms. For example, the gaseous substrate containing CO2 may be provided dissolved in a liquid. Essentially, a liquid is saturated with a carbon dioxide containing gas and then that liquid is added to the bioreactor. This may be achieved using standard methodology. By way of example, a microbubble dispersion generator (Hensirisak et. al. Scale-up of microbubble dispersion generator for aerobic fermentation; Applied Biochemistry and Biotechnology Volume 10 1, Number 3 / October, 2002) could be used. By way of further example, the gaseous substrate containing CO2 and H2 may be adsorbed onto a solid support.

The terms "increasing the efficiency", "increased efficiency" and the like, when used in relation to a fermentation process, include, but are not limited to, increasing one or more of the rate of growth of microorganisms catalysing the fermentation, the growth and/or product production rate at elevated butanediol concentrations, the volume of desired product produced per volume of substrate consumed, the rate of production or level of production of the desired product, and the relative proportion of the desired product produced compared with other by-products of the fermentation.

The terms "productivity" or "rate of production" is the volumetric productivity of a product. In continuous systems the volumetric productivity is calculated as the ratio of the steady state concentration of the product and the liquid retention time. In batch systems the volumetric productivity is calculated as the concentration and the time required to produce said concentration in a batch system. The volumetric productivity is reported as g/L/day.

Unless the context requires otherwise, the phrases "fermenting", "fermentation process" or "fermentation reaction" and the like, as used herein, are intended to encompass both the growth phase and product biosynthesis phase of the process.

The term "products derived from pyruvate" or similar terms as used herein are intended to encompass fermentation products having a pyruvate precursor. These products include, but are not limited to, 2,3-butanediol, lactate, succinate, Methyl Ethyl Ketone (MEK), 2-butanol, propanediol, 2-propanol, isopropanol, acetoin, iso-butanol, citramalate, butadiene, and poly lactic acid.

The term "Acetyl CoA derived products", "products derived from Acetyl CoA" or similar terms as used herein are intended to encompass fermentation products having an Acetyl CoA precursor. These products include but are not limited to ethanol, acetic acid, acetone, butanol, 3-hydroxybutyrate and isobutylene, 3-hydroxy propionate (3HP) and fatty acids.

It has been discovered that 2,3-butanediol production in fermentation processes increases during times when the microbial culture is exhibiting signs of stress. The inventors have identified several indicators of stress that correspond with an increase in the amount of 2,3-butandiol, including production of lactate by the microbial culture, increased pH of the microbial culture, and a decrease in the biomass concentration of the microbial culture. Interestingly, the inventors have demonstrated that the production of 2,3-butanediol by microbial culture is not an indicator of stress, and that it is possible to provide a healthy and stable microbial culture having an increased 2,3-butanediol productivity.

It has previously been shown that increased 2,3-butandiol productivity was influenced by a rate of hydrogen consumption by a microbial culture (WO2012131627).

### Effect of Co2 on Fermentation

The inventors have found that by altering the amount of CO2 provided to the microbial culture, the metabolic pathway of the microorganism is affected. By altering the amount of CO2 provided to the microbial culture, the metabolism of the culture can be manipulated.

The inventors have surprisingly shown that the production of pyruvate derived products is increased when the microbial culture is provided with an increased amount of carbon dioxide. Correspondingly it has been found that the production of products derived from Acetyl CoA is increased, and the production of pyruvate derived products is decreased when the amount of CO2 dissolved in the microbial culture is decreased.

It has been shown previously that providing a carboxydotrophic culture with a substrate comprising CO and optionally hydrogen, under fermentation conditions, results in the production of alcohols and acids. It has also been previously demonstrated the production of ethanol, with the production of additional by-products including 2,3-butanediol and acetic acid.

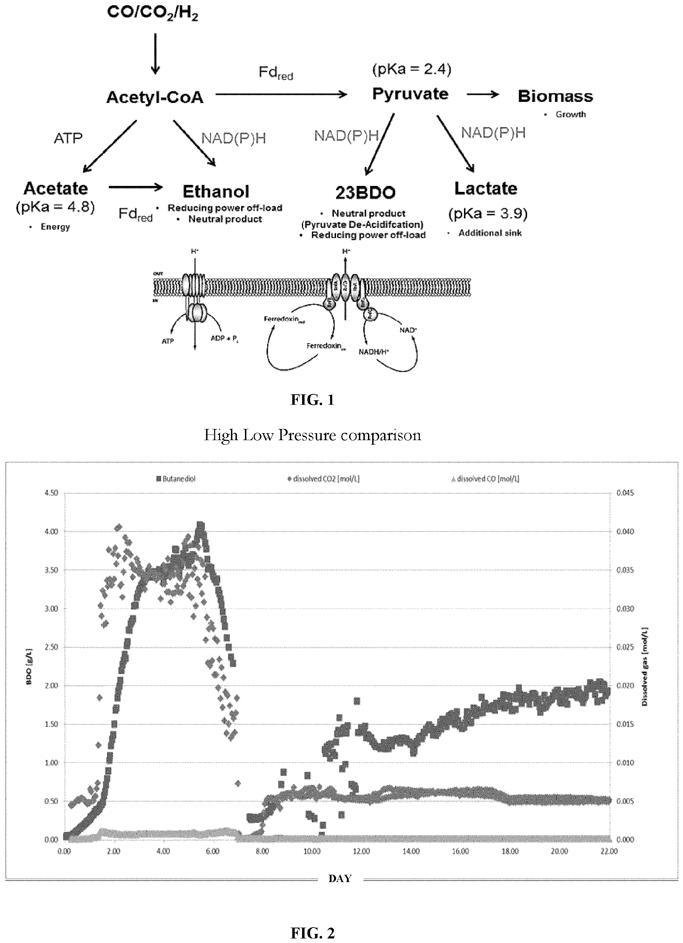

The inventors have now discovered that by additionally supplying the microbial culture with carbon dioxide, the metabolism of the pyruvate arm of the metabolic pathway can be controlled. The metabolic pathway described above is shown in more detail in Figure 1 and below.

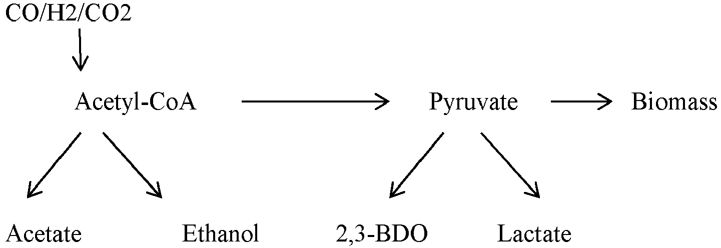

Carboxydotrophic acetogens use the Wood-Ljungdahl pathway to fix carbon into Acetyl-CoA (Drake, Küsel, Matthies, Wood, & Ljungdahl, 2006; Wood, 1991), which serves as a precursor for products such as acetate and ethanol and for fatty acid biosynthesis. Beside acetyl-CoA, the other key intermediate in the cell is Pyruvate (pyruvic acid) which serves as precursor for products like 2,3-butanediol, lactic acid, or succinic acid, as well as amino acids, vitamins, or nucleic acids required for growth and biomass formation. Acetyl-CoA can be directly converted into pyruvate or vice versa in a single, reversible enzymatic step catalyzed by a pyruvate:ferredoxin oxidoreductase (PFOR), sometimes also referred to as pyruvate synthase (EC 1.2.7.1). The PFOR reaction looks as follows in reaction 1:

(1) Acetyl-CoA + CO₂ + reduced ferredoxin + 2 H+ <-> Pyruvate + oxidized ferredoxin ΔG^{O}' = -4.6 kcal/mol (19.2 kJ/mol) (Thauer, Jungermann, Decker, & Pi, 1977)

In carboxydotrophic acetogens that grow autotrophically all produced pyruvate has to go through acetyl-CoA first. As acetyl-CoA is a C2 compound and pyruvate a C3 compound, a molecule of CO₂ needs to be incorporated (reaction 1). The energy for this reaction is provided by reduced ferredoxin (E₀' = -398 mV).

A strategy to increase the rate of pyruvate formation is to increase the level of educts or reactants in this reaction (dynamic equilibrium). For example, increasing the level of CO2 in the feed gas will increase the pyruvate formation rate from acetyl-CoA, while the reverse reaction decreases up to a point where the reaction is virtually irreversible in direction of pyruvate formation. Similarly, the level of reduced ferredoxin can be increased by, for example, increasing the rate of CO oxidation via the ferredoxin-dependent carbon monoxide dehydrogenase.

Pyruvate (pyruvic acid) is an acid with a very low pKa of 2.5 and thus at higher concentrations a threat to the bacteria by destroying the essential proton gradient across the membrane required for ATP formation (Köpke & Dürre, 2011). A sink for the bacteria is to produce 2,3-butanediol that will allow it to neutralize pyruvic acid and save the cell. Increasing the level of CO2 in the feed gas will therefore increase the 2,3-butanediol formation indirectly via increased rates of pyruvate formation. The reaction for production of 2,3-butanediol from pyruvate is as follows in reaction 2:

(2) 2 Pyruvate <-> Acetoin + 2 CO₂ Acetoin + NAD(P)H + H+ <-> 2,3-butanediol + NAD(P)+

Lactic acid and Succinic acid are pyruvate-derived products that represent another sink and although they are much weaker acids (pKa 4.2 and 5.6 respectively), they also cause a threat to the bacteria at higher levels. On the other hand, limiting their production could increase the pyruvate pool and result in increased 2,3-butanediol production.

The inventors have shown that by increasing the concentration of CO2 in the reactor and/or by increasing the concentration of CO in the reactor or the rate of CO oxidation by the CODH leading to an increased level of reduced ferredoxin, the production of pyruvate relative to acetyl-CoA can be increased.

In particular, the inventors have demonstrated that the ratio of acetyl-CoA derived products, e.g., ethanol, to pyruvate derived products, e.g., 2,3-butanediol, may be increased by increasing the concentration of CO2 dissolved in the liquid medium of the reactor. The amount of CO2 dissolved in the liquid nutrient medium may be increased by increasing the amount of CO2 in the gaseous substrate provided to the fermentation. In one embodiment the concentration of CO2 in the substrate provided to the bioreactor is at least 10%, or at least 15%, or at least 18%, or at least 20%, or at least 25%, or at least 30%, or at least 35%, or at least 40%, or at least 45%. In certain embodiments, the concentration of CO2 in the substrate provided to the bioreactor is between 15% and 65%, or from about 20% to about 50%, or from about 25% to about 45%.

While low dissolved CO2 concentrations (for example, 0 to 10% CO2 in the inlet gas stream) provided to the culture will produce ethanol to 2,3-butandiol at a ratio from about 30:1 to about 20:1, the inventors have shown that increased CO2 concentrations (for example 10-65% CO2 in the inlet gas stream) provided to the culture will produce ethanol to 2,3-butanediol ratio from about 20:1 to 1:1, preferably 10:1 to 1:1.

In instances where low production of pyruvate derived products is desired, a low dissolved CO2 concentration may be targeted. This method may also be used in order to increase the production of acetyl-CoA derived products. For example, an gas inlet stream with 0-10% CO2 in the inlet gas stream will result in a high ethanol to 2,3-butanediol ratio.

Further, it has been found that increasing the amount of CO consumed by the culture increases the amount of CO2 produced, which in turn increases the production of pyruvate derived products. The amount of CO consumed by the culture may be increased by altering mass transfer in the fermentation, increasing the rate of flow of the gaseous substrate to the bioreactor and/or by increasing a rate of agitation of the liquid nutrient medium in the bioreactor. The amount of CO consumed by the culture may also be increased by increasing a bubble surface area. Typically, high mass transfer can be achieved by introducing the gaseous substrate as fine bubbles. Those skilled in the art will appreciate means for introducing gaseous substrate, such as spargers.

### Dissolved CO2 and Pressure

The inventors have identified a number of methods for controlling and adjusting the amount of dissolved CO2 provided to a microbial culture to control the metabolic profile of the fermentation. One such method for adjusting the amount of CO2 dissolved in the liquid nutrient medium includes adjusting the pressure to the system.

The inventors have demonstrated that increasing the pressure in the bioreactor will lead to an increase in the amount of dissolved CO2 in the fermentation medium. In order to increase production of pyruvate-derived products, the fermentation should be carried out at a pressure of about 250 to about 450 kPag (or greater than 500 kPag), such that the concentration of CO2 dissolved in the liquid nutrient medium is increased. In certain embodiments, the pressure is greater than 250 kPag or greater than 300 kPag or greater than 350 kPag or greater than 400 kPag, or greater than 450 kPag or greater than 500 kPag.

In instances where the CO2 is provided to the reactor at pressure of 50 kPag or greater, a lower concentration of CO2 is required in the substrate in order to produce higher levels of pyruvate derived products. As the culture produces CO2 through utilisation of CO, an inlet gas stream with a minimal CO2 concentration may be supplied to the reactor if the pressure is substantially high. In certain embodiments the amount of CO2 provided to the reactor at a pressure of 50 kPag or higher, is less than 10%, or less than5 %, or less than 1%. In certain embodiments substantially no CO2 is provided to the reactor at a pressure of 50 kPag or greater. Preferably, the CO2 concentration of an inlet gas stream supplied at pressure of 50 kPag or greater is from about 0% to 50%.

The inventors have shown that the production of 2,3-butanediol is influenced by the amount of CO2 partial pressure in the fermenter, which in turn changes the amount of CO2 dissolved in the liquid nutrient medium. Higher CO2 partial pressures of the gas stream will increase the amount of CO2 dissolved in the liquid nutrient medium. In preferred embodiments, the CO2 will be supplied to the reactor at a partial pressure between about 50 kPag to about 500 kPag.

Furthermore, the inventors have demonstrated that it is also possible to gradually increase the amount of dissolved CO2 by gradually increasing the amount of CO2 supplied to the reactor.

The amount of CO2 in some gaseous streams may not be sufficient to enable a sufficient amount of dissolved CO2 in the liquid nutrient medium. In order to overcome this problem, the inventors have provided a method and system for increasing the amount of CO2 by recycling a tail or exit gas from the outlet of the bioreactor to the inlet of the bioreactor. In order to change the amount of CO2 partial pressure, and therefore the dissolved CO2, independently from the CO partial pressure and total pressure, the exit gas/tail gas may be recycled to the same reactor. The fermentation process within the reactor will result in high conversion of CO and H2, and therefore the tail gas will consist mainly of CO2 and any inert gas species. Thus, recycling the tail gas would allow the CO2 partial pressure to be controlled independently from the CO partial pressure and the total pressure.

The use of a two reactor system allows an exit gas comprising CO2 exiting a first bioreactor to be passed to a second bioreactor. By feeding the exit gas comprising CO2 to the downcomer of the second bioreactor, rather than to the reactor vessel, the partial pressure of CO2 in the reactor is increased. As the C02-liquid mixture travels down the downcomer, the hydrostatic head increases, thereby increasing the amount of CO2 dissolved in the solution.

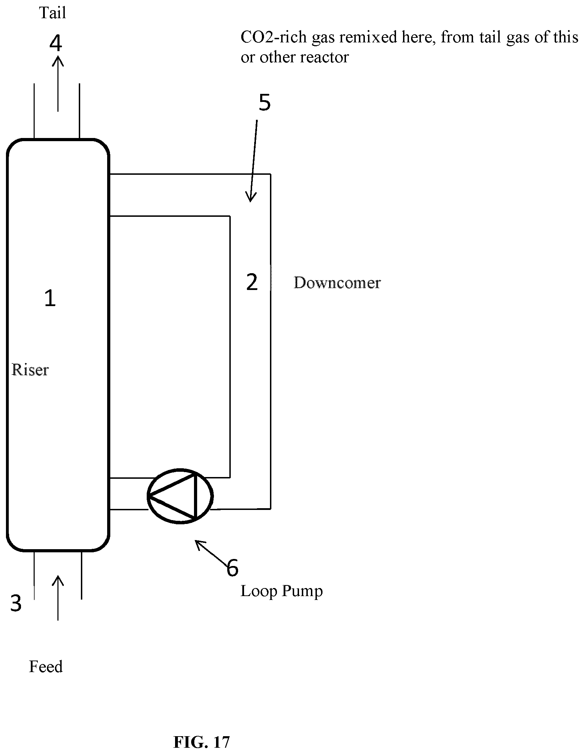

To recycle the tail gas from a first reactor to a second or receiving reactor, the headspace pressure of the first reactor must be slightly higher than the pressure at the downcomer of the receiving reactor, to overcome line loss and sparger pressure drop. To recycle "tail gas" from its own headspace, the tail gas could either be recycled to the gas inlet or the downcomer, wherein the downcomer would need an eductor to capture the tail gas (using the liquid flow in the downcomer to entrain the tail gas). The amount of CO2 being recycled into the downcomer would be controlled so that the CO2 dissolved in the liquid nutrient medium would be optimized during ramping. Figure 17 provides a representation of a circulated loop reactor with a CO2-rich substrate provided to the downcomer, wherein (1) is the riser; (2) is the downcomer; (3) is the feed gas; (4) is the tail/exit gas; (5) is the point where CO2-rich gas from the tail gas of either a separate reactor or the same reactor enters the downcomer; and (6) is the loop pump which circulates the gas/liquid mixture through the riser and downcomer.

### The bioreactor

The fermentation may be carried out in any suitable bioreactor, such as a continuous stirred tank reactor (CSTR), an immobilised cell reactor, a gas-lift reactor, a bubble column reactor (BCR), a membrane reactor, such as a Hollow Fibre Membrane Bioreactor (HFM BR) or a trickle bed reactor (TBR). Also, in some embodiments of the invention, the bioreactor may comprise a first, growth reactor in which the micro-organisms are cultured, and a second, fermentation reactor, to which fermentation broth from the growth reactor may be fed and in which most of the fermentation product (e.g. ethanol and acetate) may be produced. The bioreactor of the present invention is adapted to receive a CO and/or H₂ containing substrate.

### The fermentation substrate

A substrate comprising carbon monoxide and at least one of hydrogen or carbon dioxide, is used in the fermentation reaction to produce one or more products in the methods of the invention. Preferably the substrate is a gaseous substrate. The gaseous substrate may be a waste gas obtained as a by-product of an industrial process, or from some other source such as from combustion engine (for example automobile) exhaust fumes. In certain embodiments, the industrial process is selected from the group consisting of ferrous metal products manufacturing, such as a steel mill, non-ferrous products manufacturing, petroleum refining processes, gasification of coal, electric power production, carbon black production, ammonia production, methanol production, coke manufacturing and natural gas reforming. In these embodiments, the gaseous substrate may be captured from the industrial process before it is emitted into the atmosphere, using any convenient method. Depending on the composition of the gaseous substrate, it may also be desirable to treat it to remove any undesired impurities, such as dust particles before introducing it to the fermentation. For example, the gaseous substrate may be filtered or scrubbed using known methods.

In other embodiments of the invention, the gaseous substrate may be sourced from the gasification of biomass. The process of gasification involves partial combustion of biomass in a restricted supply of air or oxygen. The resultant gas typically comprises mainly CO and H₂, with minimal volumes of CO₂, methane, ethylene and ethane. For example, biomass by-products obtained during the extraction and processing of foodstuffs such as sugar from sugarcane, or starch from maize or grains, or non-food biomass waste generated by the forestry industry may be gasified to produce a CO-containing gas suitable for use in the present invention.

The CO-containing substrate will typically contain a major proportion of CO, such as at least about 15% to about 100% CO by volume, from 40% to 95% CO by volume, from 40% to 60% CO by volume, and from 45% to 55% CO by volume. In particular embodiments, the substrate comprises about 25%, or about 30%, or about 35%, or about 40%, or about 45%, or about 50% CO, or about 55% CO, or about 60% CO by volume. Substrates having lower concentrations of CO, such as 6%, may also be appropriate, particularly when H₂ and CO₂ are also present.

Typically, the carbon monoxide will be added to the fermentation reaction in a gaseous state. However, the invention should not be considered to be limited to addition of the substrate in this state. For example, the carbon monoxide could be provided in a liquid. For example, a liquid may be saturated with a carbon monoxide containing gas and then that liquid added to a bioreactor. This may be achieved using standard methodology. By way of example, a microbubble dispersion generator as described above can be used.

In one embodiment the carbon dioxide is added to the fermentation in a gaseous state. In alternative embodiment, the carbon dioxide is provided as a carbonate or bicarbonate.

In one embodiment of the invention, a combination of two or more different substrates may be used in the fermentation reaction.

In addition, it is often desirable to increase the CO concentration of a substrate stream (or CO partial pressure in a gaseous substrate) and thus increase the efficiency of fermentation reactions where CO is a substrate. Increasing CO partial pressure in a gaseous substrate increases CO mass transfer into a fermentation media. The composition of gas streams used to feed a fermentation reaction can have a significant impact on the efficiency and/or costs of that reaction. For example, O2 may reduce the efficiency of an anaerobic fermentation process. Processing of unwanted or unnecessary gases in stages of a fermentation process before or after fermentation can increase the burden on such stages (e.g. where the gas stream is compressed before entering a bioreactor, unnecessary energy may be used to compress gases that are not needed in the fermentation). Accordingly, it may be desirable to treat substrate streams, particularly substrate streams derived from industrial sources, to remove unwanted components and increase the concentration of desirable components.

In certain embodiments, little or no hydrogen is provided in the CO comprising substrate.

### Blending of Streams

It may be desirable to blend a reformed substrate stream comprising CO and H2 with one or more further streams in order to improve efficiency, alcohol production and/or overall carbon capture of the fermentation reaction. Without wishing to be bound by theory, in some embodiments of the present invention, carboxydotrophic bacteria convert CO to ethanol according to the following:

6CO + 3H₂O → C₂H₅OH + 4CO₂

However, in the presence of H2, the overall conversion can be as follows:

6CO + 12H₂ → 3C₂H₅OH + 3H₂O

Accordingly, streams with high CO content can be blended with reformed substrate streams comprising CO and H2 to increase the CO:H2 ratio to optimise fermentation efficiency. By way of example, industrial waste streams, such as off-gas from a steel mill have a high CO content, but include minimal or no H2. As such, it can be desirable to blend one or more streams comprising CO and H2 with the waste stream comprising CO, prior to providing the blended substrate stream to the fermenter. The overall efficiency, alcohol productivity and/or overall carbon capture of the fermentation will be dependent on the stoichiometry of the CO and H2 in the blended stream. However, in particular embodiments the blended stream may substantially comprise CO and H2 in the following molar ratios: 20:1, 10:1, 5:1, 3:1, 2:1, 1:1 or 1:2.

In addition, it may be desirable to provide CO and H2 in particular ratios at different stages of the fermentation. For example, substrate streams with a relatively high H2 content (such as 1:2 CO:H2) may be provided to the fermentation stage during start up and/or phases of rapid microbial growth. However, when the growth phase slows, such that the culture is maintained at a substantially steady microbial density, the CO content may be increased (such as at least 1:1 or 2:1 or higher, wherein the H2 concentration may be greater or equal to zero).

Blending of streams may also have further advantages, particularly in instances where a waste stream comprising CO is intermittent in nature. For example, an intermittent waste stream comprising CO may be blended with a substantially continuous reformed substrate stream comprising CO and H2 and provided to the fermenter. In particular embodiments of the invention, the composition and flow rate of the substantially continuous blended stream may be varied in accordance with the intermittent stream in order to maintain provision of a substrate stream of substantially continuous composition and flow rate to the fermenter.

### Media

It will be appreciated that for growth of the one or more microorganisms and substrate to ethanol and/or acetate fermentation to occur, in addition to the substrate, a suitable nutrient medium will need to be fed to the bioreactor. A nutrient medium will contain components, such as vitamins and minerals, sufficient to permit growth of the micro-organism used. By way of example only, anaerobic media suitable for the growth of *Clostridium autoethanogenum* are known in the art, as described for example by Abrini et al (Clostridium autoethanogenum, sp. Nov., An Anaerobic Bacterium That Produces Ethanol From Carbon Monoxide; Arch. Microbiol., 161: 345-351 (1994)). The "Examples" section herein after provides further examples of suitable media.

### Fermentation

Processes for the production of ethanol and other alcohols from gaseous substrates are known. Exemplary processes include those described for example in WO2007/117157, WO2008/115080, WO2009/022925, WO2009/064200, US 6,340,581, US 6,136,577, US 5,593,886, US 5,807,722 and US 5,821,111, each of which is incorporated herein by reference.

### Fermentation conditions

The fermentation should desirably be carried out under appropriate conditions for the substrate to ethanol and/or acetate fermentation to occur. Reaction conditions that should be considered include temperature, media flow rate, pH, media redox potential, agitation rate (if using a continuous stirred tank reactor), inoculum level, maximum substrate concentrations and rates of introduction of the substrate to the bioreactor to ensure that substrate level does not become limiting, and maximum product concentrations to avoid product inhibition.

The optimum reaction conditions will depend partly on the particular microorganism of used. However, in general, it is preferred that the fermentation be performed at a pressure higher than ambient pressure. Operating at increased pressures allows a significant increase in the rate of CO transfer from the gas phase to the liquid phase where it can be taken up by the micro-organism as a carbon source for the production of ethanol. This in turn means that the retention time (defined as the liquid volume in the bioreactor divided by the input gas flow rate) can be reduced when bioreactors are maintained at elevated pressure rather than atmospheric pressure.

Also, since a given CO-to-product conversion rate is in part a function of the substrate retention time, and achieving a desired retention time in turn dictates the required volume of a bioreactor, the use of pressurized systems can greatly reduce the volume of the bioreactor required, and consequently the capital cost of the fermentation equipment. According to examples given in US patent no. 5,593,886, reactor volume can be reduced in linear proportion to increases in reactor operating pressure, i.e. bioreactors operated at 10 atmospheres of pressure need only be one tenth the volume of those operated at 1 atmosphere of pressure.

The benefits of conducting a gas-to-product fermentation at elevated pressures have also been described elsewhere. For example, WO 02/08438 describes gas-to-ethanol fermentations performed under pressures of 30 psig and 75 psig, giving ethanol productivities of 150 g/l/day and 369 g/l/day respectively. However, example fermentations performed using similar media and input gas compositions at atmospheric pressure were found to produce between 10 and 20 times less ethanol per litre per day.

Examples of fermentation conditions suitable for anaerobic fermentation of a substrate comprising CO are detailed in WO2007/117157, WO2008/115080, WO2009/022925 and WO2009/064200. It is recognised the fermentation conditions reported therein can be readily modified in accordance with the methods of the instant invention.

### Microorganisms

In various embodiments, the fermentation is carried out using a culture of one or more strains of carboxydotrophic bacteria. In various embodiments, the carboxydotrophic bacterium is selected from *Moorella, Clostridium, Ruminococcus, Acetobacterium, Eubacterium, Butyribacterium, Oxobacter, Methanosarcina, Methanosarcina,* and *Desulfotomaculum.* A number of anaerobic bacteria are known to be capable of carrying out the fermentation of CO to alcohols, including n-butanol and ethanol, and acetic acid, and are suitable for use in the process of the present invention. Examples of such bacteria that are suitable for use in the invention include those of the genus *Clostridium,* such as strains of *Clostridium ljungdahlii,* including those described in WO 00/68407, EP 117309, US patent No's 5,173,429, 5,593,886, and 6,368,819, WO 98/00558 and WO 02/08438, *Clostridium carboxydivorans* (Liou et al., International Journal of Systematic and Evolutionary Microbiology 33: pp 2085-2091), *Clostridium ragsdalei* (WO/2008/028055) and *Clostridium autoethanogenum* (Abrini et al, Archives of Microbiology 161: pp 345-351). Other suitable bacteria include those of the genus *Moorella,* including *Moorella sp* HUC22-1, (Sakai et al, Biotechnology Letters 29: pp 1607-1612), and those of the genus *Carboxydothermus* (Svetlichny, V.A., Sokolova, T.G. et al (1991), Systematic and Applied Microbiology 14: 254-260). Further examples include *Moorella thermoacetica, Moorella thermoautotrophica, Ruminococcus productus, Acetobacterium woodii, Eubacterium limosum, Butyribacterium methylotrophicum, Oxobacter pfennigii, Methanosarcina barkeri, Methanosarcina acetivorans, Desulfotomaculum kuznetsovii* (Simpa et. al. Critical Reviews in Biotechnology, 2006 Vol. 26. Pp41-65). In addition, it should be understood that other acetogenic anaerobic bacteria may be applicable to the present invention as would be understood by a person of skill in the art. It will also be appreciated that the invention may be applied to a mixed culture of two or more bacteria.

In one embodiment, the microorganism is selected from the group of acetogenic carboxydotrophic organisms comprising the species *Clostridium autoethanogenum, Clostridium Ijungdahlii, Clostridium ragsdalei, Clostridium carboxidivorans, Clostridium drakei, Clostridium scatologenes, Clostridium aceticum, Clostridium formicoaceticum, Clostridium magnum, Acetobacterium woodii, Alkalibaculum bacchii, Moorella thermoacetica, Sporomusa ovate, Butyribacterium methylotrophicum, Blautia producta, Eubacterium limosum, Thermoanaerobacter kiuvi.*

These carboxydotrophic acetogens are defined by their ability to utilize and grow chemoautotrophically on gaseous one-carbon (C1) sources such as carbon monoxide (CO) and carbon dioxide (CO2) with carbon monoxide (CO) and/or hydrogen (H2) as energy source under anaerobic conditions forming acetyl-CoA, acetate and other products. They share the same mode of fermentation, the Wood-Ljungdahl or reductive acetyl-CoA pathway, and are defined by the presence of the enzyme set consisting of Carbon monoxide dehydrogenase (CODH), Hydrogenase, Formate dehydrogenase, Formyl-tetrahydrofolate synthetase, Methylene-tetrahydrofolate dehydrogenase, Formyl-tetrahydrofolate cyclohydrolase, Methylene-tetrahydrofolate reductase, and Carbon monoxide dehydrogenase/Acetyl-CoA synthase (CODH/ACS), which combination is characteristic and unique to this type of bacteria (Drake, Küsel, Matthies, Wood, & Ljungdahl, 2006).

In contrast to chemoheterotrophic growth of sugar-fermenting bacteria that convert the substrate into biomass, secondary metabolites and pyruvate from which then products are formed (either via acetyl-CoA or directly), in acetogens the substrate is channelled directly into acetyl-CoA, from which then products, biomass, and secondary metabolites are formed.

In a further embodiment, the microorganism is selected from a cluster of carboxydotrophic Clostridia comprising the species *C*. *autoethanogenum, C. ljungdahlii,* and "*C*. *ragsdalei"* and related isolates.

These include but are not limited to strains *C*. *autoethanogenum* JAI-1^{T} (DSM10061) (Abrini, Naveau, & Nyns, 1994), *C. autoethanogenum* LBS1560 (DSM19630) (WO/2009/064200), *C*. *autoethanogenum* LBS1561 (DSM23693), *C*. *ljungdahlii* PETC^{T} (DSM13528 = ATCC 55383) (Tanner, Miller, & Yang, 1993), C. *Ijungdahlii* ERI-2 (ATCC 55380) (US patent 5,593,886), C. *Ijungdahlii* C-01 (ATCC 55988) (US patent 6,368,819), C. *Ijungdahlii* O-52 (ATCC 55989) (US patent 6,368,819), or "*C*. *ragsdalei* P11^{T}" (ATCC BAA-622) (WO 2008/028055), and related isolates such as "*C*. *coskatii"* (US patent 2011/0229947), *"Clostridium sp.* MT351 " (Tyurin & Kiriukhin, 2012), *"Clostridium sp.* MT 653 "(Berzin, Kiriukhin, & Tyurin, 2012a), *"Clostridium sp.* MT683 " (Berzin, 2012), *"Clostridium sp.* MT962" (Berzin, Kiriukhin, & Tyurin, 2013) *"Clostridium sp.* MT1121" (Berzin, Kiriukhin, & Tyurin, 2012b), *"Clostridium sp.* MT1230" (Kiriukhin & Tyurin, 2013), or *"Clostridium sp.* MT1962" (Berzin, Tyurin, & Kiriukhin, 2013), and mutant strains thereof such as C. *ljungdahlii* OTA-1 (Tirado-Acevedo O. Production of Bioethanol from Synthesis Gas Using *Clostridium ljungdahlii.* PhD thesis, North Carolina State University, 2010) or *"Clostridium sp.* MT896" (Berzin, Kiriukhin, & Tyurin, 2012c).

These strains form a subcluster within the Clostridial rRNA cluster I (Collins et al., 1994), having at least 99% identity on 16S rRNA gene level, although being distinct species as determined by DNA-DNA reassociation and DNA fingerprinting experiments (WO 2008/028055, US patent 2011/0229947).

The strains of this cluster are defined by common characteristics, having both a similar genotype and phenotype, and they all share the same mode of energy conservation and fermentative metabolism. The strains of this cluster lack cytochromes and conserve energy via an Rnf complex.

All strains of this cluster have a similar genotype with a genome size of around 4.2 MBp (Köpke et al., 2010) and a GC composition of around 32 %mol (Abrini et al., 1994; Köpke et al., 2010; Tanner et al., 1993) (WO 2008/028055; US patent 2011/0229947), and conserved essential key gene operons encoding for enzymes of Wood-Ljungdahl pathway (Carbon monoxide dehydrogenase, Formyl-tetrahydrofolate synthetase, Methylene-tetrahydrofolate dehydrogenase, Formyl-tetrahydrofolate cyclohydrolase, Methylene-tetrahydrofolate reductase, and Carbon monoxide dehydrogenase/Acetyl-CoA synthase), hydrogenase, formate dehydrogenase, Rnf complex (*rnfCDGEAB*)*,* pyruvate:ferredoxin oxidoreductase, aldehyde:ferredoxin oxidoreductase (Köpke et al., 2010, 2011). The organization and number of Wood-Ljungdahl pathway genes, responsible for gas uptake, has been found to be the same in all species, despite differences in nucleic and amino acid sequences (Köpke et al., 2011).

The strains all have a similar morphology and size (logarithmic growing cells are between 0.5-0.7 x 3-5 µm), are mesophilic (optimal growth temperature between 30-37 °C) and strictly anaerobe (Abrini et al., 1994; Tanner et al., 1993)(WO 2008/028055). Moreover, they all share the same major phylogenetic traits, such as same pH range (pH 4-7.5, with an optimal initial pH of 5.5-6), strong autotrophic growth on CO containing gases with similar growth rates, and a similar metabolic profile with ethanol and acetic acid as main fermentation end product, and small amounts of 2,3-butanediol and lactic acid formed under certain conditions(Abrini et al., 1994; Köpke et al., 2011; Tanner et al., 1993)(WO 2008/028055). Indole production was observed with all species. However, the species differentiate in substrate utilization of various sugars (e.g. rhamnose, arabinose), acids (e.g. gluconate, citrate), amino acids (e.g. arginine, histidine), or other substrates (e.g. betaine, butanol). Moreover some of the species were found to be auxotroph to certain vitamins (e.g. thiamine, biotin) while others were not. Also reduction of carboxylic acids into their corresponding alcohols has been shown in a range of these organisms (Perez, Richter, Loftus, & Angenent, 2012). These traits are therefore not specific to one organism like *C*. *autoethanogenum* or C. *Ijungdahlii,* but rather general traits for carboxydotrophic, ethanol-synthesizing Clostridia and it can be anticipated that mechanism work similar across these strains, although there may be differences in performance (Perez et al., 2012).

One exemplary micro-organism suitable for use in the present invention is *Clostridium autoethanogenum.* In one embodiment, the *Clostridium autoethanogenum* is a *Clostridium autoethanogenum* having the identifying characteristics of the strain deposited at the German Resource Centre for Biological Material (DSMZ) under the identifying deposit number 19630. In another embodiment, the *Clostridium autoethanogenum* is a *Clostridium autoethanogenum* having the identifying characteristics of DSMZ deposit number DSMZ 10061. These strains have a particular tolerance to changes in substrate composition, particularly of H₂ and CO and as such are particularly well suited for use in combination with a steam reforming process.

One exemplary micro-organism suitable for use in the production of acetate from a substrate comprising CO2 and H2 in accordance with one aspect of the present invention is *Acetobacterium woodii.*

Culturing of the bacteria used in the methods of the invention may be conducted using any number of processes known in the art for culturing and fermenting substrates using anaerobic bacteria. By way of example, those processes generally described in the following articles using gaseous substrates for fermentation may be utilised: (i) K. T. Klasson, et al. (1991). Bioreactors for synthesis gas fermentations resources. Conservation and Recycling, 5; 145-165; (ii) K. T. Klasson, et al. (1991). Bioreactor design for synthesis gas fermentations. Fuel. 70. 605-614; (iii) K. T. Klasson, et al. (1992). Bioconversion of synthesis gas into liquid or gaseous fuels. Enzyme and Microbial Technology. 14; 602-608; (iv) J. L. Vega, et al. (1989). Study of Gaseous Substrate Fermentation: Carbon Monoxide Conversion to Acetate. 2. Continuous Culture. Biotech. Bioeng. 34. 6. 785-793; (v) J. L. Vega, et al. (1989). Study of gaseous substrate fermentations: Carbon monoxide conversion to acetate. 1. Batch culture. Biotechnology and Bioengineering. 34. 6. 774-784; (vi) J. L. Vega, et al. (1990). Design of Bioreactors for Coal Synthesis Gas Fermentations. Resources, Conservation and Recycling. 3. 149-160; all of which are incorporated herein by reference.

### Fermentation products

Methods of the invention can be used to produce any of a variety of hydrocarbon products. This includes alcohols, acids and/or diols. More particularly, the invention may be applicable to fermentation to produce butyrate, propionate, caproate, ethanol, propanol, butanol, 2,3-butanediol, propylene, butadiene, iso-butylene and ethylene. In one embodiment the invention can be used to produce alcohols including but not limited to propanol and butanol. The alcohol(s) can then be reacted with acetate to produce product(s) including propyl acetate or butyl acetate. A skilled person would understand that the invention is not limited to the alcohols and products mentioned, any appropriate alcohol and or acid can be used to produce a product.

These and other products may be of value for a host of other processes such as the production of plastics, pharmaceuticals and agrochemicals. In one embodiment, the fermentation product is used to produce gasoline range hydrocarbons (about 8 carbon), diesel hydrocarbons (about 12 carbon) or jet fuel hydrocarbons (about 12 carbon).

The methods of the invention can also be applied to aerobic fermentations, to anaerobic or aerobic fermentations of other products, including but not limited to isopropanol. The methods of the invention can also be applied to aerobic fermentations, and to anaerobic or aerobic fermentations of other products, including but not limited to isopropanol.

The invention also provides that at least a portion of a hydrocarbon product produced by the fermentation is reused in the steam reforming process. This may be performed because hydrocarbons other than CH₄ are able to react with steam over a catalyst to produce H₂ and CO. In a particular embodiment, ethanol is recycled to be used as a feedstock for the steam reforming process. In a further embodiment, the hydrocarbon feedstock and/or product is passed through a prereformer prior to being used in the steam reforming process. Passing through a prereformer partially completes the steam reforming step of the steam reforming process which can increase the efficiency of hydrogen production and reduce the required capacity of the steam reforming furnace.

The methods of the invention can also be applied to aerobic fermentations, and to anaerobic or aerobic fermentations of other products, including but not limited to isopropanol.

More particularly, the invention may be applicable to fermentation to ethanol and/or acetate. These products may then be reacted to together to produce chemical products including esters. In one embodiment of the invention the ethanol and acetate produced by fermentation are reacted together to produce Ethyl Acetate. Ethyl acetate may be of value for a host of other processes such as the production of solvents including surface coating and thinners as well as in the manufacture of pharmaceuticals and flavours and essences.

### Product recovery

The products of the fermentation reaction can be recovered using known methods. Exemplary methods include those described in WO07/117157, WO08/115080, US 6,340,581, US 6,136,577, US 5,593,886, US 5,807,722 and US 5,821,111. However, briefly and by way of example ethanol may be recovered from the fermentation broth by methods such as fractional distillation or evaporation, and extractive fermentation.

Distillation of ethanol from a fermentation broth yields an azeotropic mixture of ethanol and water (i.e., 95% ethanol and 5% water). Anhydrous ethanol can subsequently be obtained through the use of molecular sieve ethanol dehydration technology, which is also well known in the art.

Extractive fermentation procedures involve the use of a water-miscible solvent that presents a low toxicity risk to the fermentation organism, to recover the ethanol from the dilute fermentation broth. For example, oleyl alcohol is a solvent that may be used in this type of extraction process. Oleyl alcohol is continuously introduced into a fermenter, whereupon this solvent rises forming a layer at the top of the fermenter which is continuously extracted and fed through a centrifuge. Water and cells are then readily separated from the oleyl alcohol and returned to the fermenter while the ethanol-laden solvent is fed into a flash vaporization unit. Most of the ethanol is vaporized and condensed while the oleyl alcohol is non-volatile and is recovered for re-use in the fermentation.

Acetate, which may be produced as a by-product in the fermentation reaction, may also be recovered from the fermentation broth using methods known in the art.

For example, an adsorption system involving an activated charcoal filter may be used. In this case, it is preferred that microbial cells are first removed from the fermentation broth using a suitable separation unit. Numerous filtration-based methods of generating a cell free fermentation broth for product recovery are known in the art. The cell free ethanol - and acetate - containing permeate is then passed through a column containing activated charcoal to adsorb the acetate. Acetate in the acid form (acetic acid) rather than the salt (acetate) form is more readily adsorbed by activated charcoal. It is therefore preferred that the pH of the fermentation broth is reduced to less than about 3 before it is passed through the activated charcoal column, to convert the majority of the acetate to the acetic acid form.

Acetic acid adsorbed to the activated charcoal may be recovered by elution using methods known in the art. For example, ethanol may be used to elute the bound acetate. In certain embodiments, ethanol produced by the fermentation process itself may be used to elute the acetate. Because the boiling point of ethanol is 78.8 °C and that of acetic acid is 107 °C, ethanol and acetate can readily be separated from each other using a volatility-based method such as distillation.

Other methods for recovering acetate from a fermentation broth are also known in the art and may be used. For example, US patent No's 6,368,819 and 6,753,170 describe a solvent and cosolvent system that can be used for extraction of acetic acid from fermentation broths. As with the example of the oleyl alcohol-based system described for the extractive fermentation of ethanol, the systems described in US patent No's 6,368,819 and 6,753,170 describe a water immiscible solvent/co-solvent that can be mixed with the fermentation broth in either the presence or absence of the fermented micro-organisms in order to extract the acetic acid product. The solvent/co-solvent containing the acetic acid product is then separated from the broth by distillation. A second distillation step may then be used to purify the acetic acid from the solvent/co-solvent system.

The products of the fermentation reaction (for example ethanol and acetate) may be recovered from the fermentation broth by continuously removing a portion of the broth from the fermentation bioreactor, separating microbial cells from the broth (conveniently by filtration), and recovering one or more product from the broth simultaneously or sequentially. In the case of ethanol it may be conveniently recovered by distillation, and acetate may be recovered by adsorption on activated charcoal, using the methods described above. The separated microbial cells are preferably returned to the fermentation bioreactor. The cell free permeate remaining after the ethanol and acetate have been removed is also preferably returned to the fermentation bioreactor. Additional nutrients (such as B vitamins) may be added to the cell free permeate to replenish the nutrient medium before it is returned to the bioreactor. Also, if the pH of the broth was adjusted as described above to enhance adsorption of acetic acid to the activated charcoal, the pH should be re-adjusted to a similar pH to that of the broth in the fermentation bioreactor, before being returned to the bioreactor.

Biomass recovered from the bioreactor may undergo anaerobic digestion in a digestion.to produce a biomass product, preferably methane. This biomass product may be used as a feedstock for the steam reforming process or used to produce supplemental heat to drive one or more of the reactions defined herein.

### EXAMPLES

The invention will now be further explained by way of the following examples.

### Example 1: Microarray Experiments

### Fermentation

Fermentations with *C*. *autoethanogenum* DSM23693 were carried out in 1.5L bioreactors at 37°C and CO-containing as sole energy and carbon source as described below. A defined liquid medium containing per litre: MgCl, CaCl₂ ( 2mM), KCl (25mM), H₃PO₄ (5mM), Fe (100µM), Ni, Zn (5µM), Mn, B, W, Mo, Se(2 µM) was used for culture growth. The medium was transferred into the bioreactor and was supplemented with a B vitamin solution and reduced with 0.2 mM Cr (II) solution. To achieve anaerobicity the reactor vessel was sparged with nitrogen. Prior to inoculation, the gas was switched to a gas mixture containing 30 % CO and 70% N2, feeding continuously to the reactor. The gas flow was initially set at 100 ml/min and the agitation was set at 300 rpm. Na₂S was dosed into the bioreactor at 0.3 ml/hr. The agitation was increased to 900 rpm at 50 rpm intervals during the growth phase of the fermentation. After 0.8 day in the batch mode, the bioreactor was switched to a continuous mode at a liquid rate of 1.8 ml/min (Dilution rate 1.7 d⁻¹). The gas flow was subsequently adjusted to reach 4 mol/L/d of CO uptake. The maximum gas flow was 435 ml/L per fermenter volume. After reaching steady stage the experiment were started by varying the CO2 concentration from 0 % to 25 %. To avoid any change in CO uptake, the CO flow and the total gas flow was kept constant while adjusting the CO2 and N2 flows relative to each other. The gas composition was only switched once 95% of the metabolites from the previous feeding regime were washed out and the metabolites had been stabilized again at a new level for at least two days such that data could be extracted for analyses. Media samples were taken to measure the biomass and metabolites and a headspace analysis of the in- and outflowing gas was performed on a regular basis.

### Sampling Procedure

Samples were collected from the bioreactor using pre-chilled tubes; the amount of sample collected was equivalent to OD 2, measured at 600nm. Three samples were collected from the bioreactor for Microarray analysis to compare gas composition and time effect over gene expression profile regarding different EtOH:BDO ratios. The first sample was collected from a gas mix of CO 30% and N2 70% and an EtOH:BDO ratio of 23:1 present in the reactor. The second sample was collected from a gas mix of CO 30%, N2 40% and CO2 30% with EtOH:BDO ratio of 13:1, this sample was collected 7hrs after the gas composition was modified. The third sample was collected from the same gas mix as the second sample, but with an EtOH:BDO ratio of 4:1, this sample was collected 3 days after addition of CO2 into the gas composition. After collection, the samples were centrifuged at 4000 RPM for 10min at 4°C and the supernatant was removed, subsequently, the pellet was snap frozen in liquid N2 and stored at -80°C until use.

### RNA extraction

After retrieval of samples from -80°C, the samples were extracted using RiboPure^{™}-Bacteria Kit (Ambion, Part Number AM1925).

### Microarray Analysis

Microarray analysis was performed by Roche using standard techniques

### Example 2: The Effects of Pressure on Fermentation

Figure 2, Figure 3 and Figure 4 show results from fermentations run at both low and high pressure, to demonstrate the effects on both the amount of dissolved CO2 present in the fermentation broth, and the concentration of metabolites produced by the fermentation. In each of these experiments a bioreactor containing a liquid nutrient medium was inoculated with a culture of *Clostridium autoethanogenum.* A gaseous substrate comprising CO and CO2 was provided to the bioreactor.

Figure 2 shows results from a first experiment, wherein the fermentation was run at different pressures, to determine the effect of pressure on the amount of dissolved CO2 and on the concentration of 2,3-butanediol (2,3-BDO) produced in the reactor.

Figure 2 shows that at high pressure from days 0-6 (320 kPag in the headspace of the reactor, and about 420 kPag at the bottom of the reactor) both the amount of dissolved CO2 in the fermentation broth, and the concentration of 2,3-BDO produced increased. When the fermentation was operated at low pressure from days 6-22 (50 kPag in the headspace, and about 150 kPag at the bottom) both the amount of dissolved CO2 in the fermentation broth and the concentration of 2,3-BDO decreased.

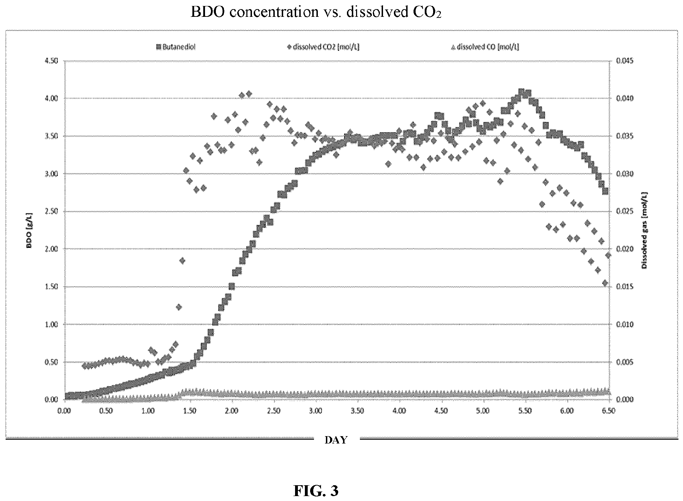

Figure 3 clearly demonstrates the correlation between the amount of dissolved CO2 in the fermentation broth and the 2,3-butanediol concentration.

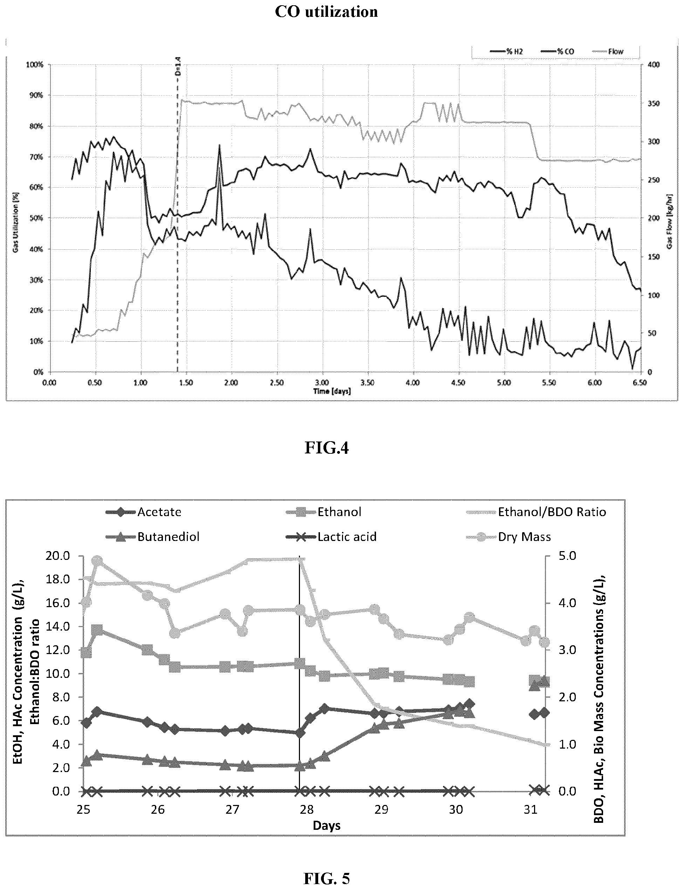

Figure 4 demonstrates the effect of CO conversion to CO2 on the fermentation. When the fermentation was operated such that the amount of CO consumed by the bacteria was increased, CO2 was produced as a by-product of the fermentation. The conversion of CO to CO2 by CODH (carbon dioxide dehydrogenase) created reduced ferredoxin. High levels of reduced ferredoxin are required to convert acetyl CoA to pyruvate, which resulted in an increase in 2,3-butanediol production, and an increase in production of other products derived from pyruvate.

### Example 3: Increasing dissolved CO2 concentrations.

A set of experiments was performed which demonstrated that the level of dissolved CO2 in the fermentation resulted in increased production of 2,3-butanediol.

### 3A: Changes in CO₂ inlet concentration as a way of increasing 2, 3 BDO production

During this experiment the CO₂ concentration of the inlet gas to the fermentation broth was changed from 0 % to 25 % in one step after 28 days of operation. The CO uptake was kept constant for the entire experiment and the concentration of CO was kept at 30 % in the inlet gas. As shown in Figure 5 a large increase in 2, 3 butanediol production was observed when the CO₂ was changed from 0% to 25%.

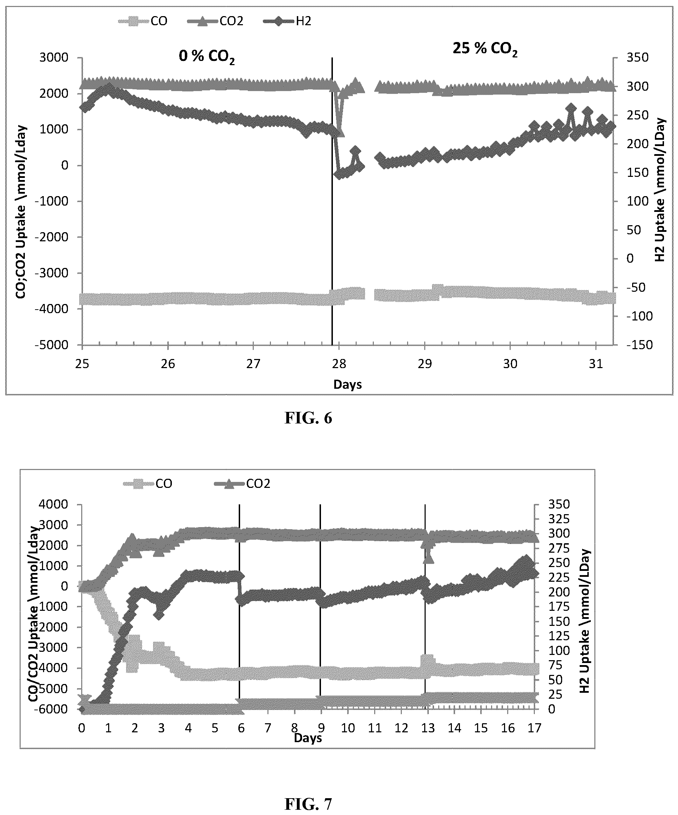

Figure 6 depicts the changes in the CO2 concentration in the fermentation broth between days 25-31. At day 25 the amount of CO2 in the inlet stream provided to the fermentation was 0%. At day 28 the CO2 concentration of the inlet stream was increased to 25%. Figure 6 clearly shows that that CO uptake stayed the same following the CO2 increase, which indicates that the increase in BDO production detailed below cannot be explained by more carbon entering the system. Further, CO2 production stayed the same after the increase. Figure 5 clearly demonstrates corresponding changes in the metabolite production of the fermentation. When CO2 was added to the fermentation broth the 2,3-BDO concentration increased from a concentration of around 0.6g/L at day 28 to 2.0 g/L at day 31. The ethanol concentration decreased, and the ethanol to 2,3-BDO ratio dropped from approximately 20:1 at day 20 to approximately 5:1 at day 31.

### 3B: High CO2 inlet concentration at start up

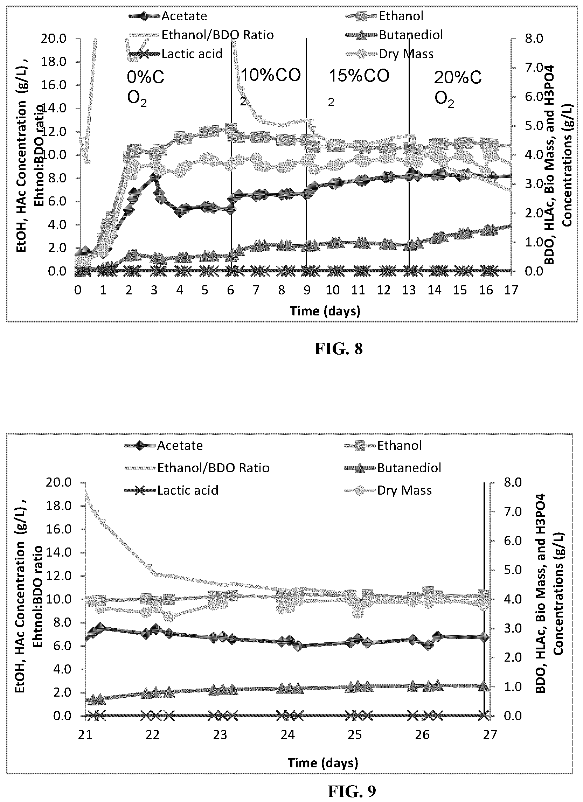

This experiment was designed to show the impact of high CO2 concentration on the production of 2,3-BDO when CO₂ was present at the beginning of the fermentation. As shown in Figure 7, once stable operating conditions were reached there was a significant 2,3-BDO production with the ethanol: 2,3-BDO ratio at 2:1. The average inlet CO2 concentration was 42 % and the average outlet CO2 concentration was 67.4 %. Throughout the experiment 50 % CO was used and the gas flow and CO uptake were adjusted to maximize ethanol and 2,3-BDO production. The concentration of CO, CO₂ and H₂ in the exit gas stream over several days is shown in Figure 8.

### 3C: Gradual increase in CO2 inlet concentration

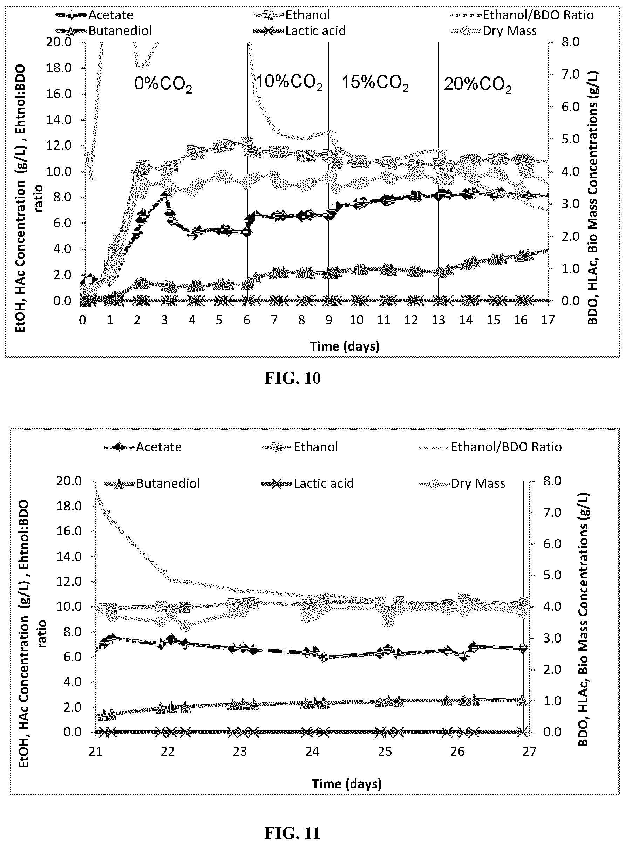

Over the duration of this experiment the concentration of CO2 in the inlet gas stream to the fermentation broth was incrementally increased to determine the effect of CO2 on the metabolite production profile of the fermentation. Figure 10 shows the effect of the increase of CO₂ in the inlet stream on metabolite concentrations. The CO2 concentration was increased from 0% to 10% at day 6; from 10% to 15% at day 9, and from 15% to 20% at day 13. At each increase in CO2 concentration in the inlet stream a corresponding increase in 2,3-BDO concentration was observed. Figure 9 shows the uptake of CO, CO₂ and H₂ of the microbial culture over the duration of the experiment.

### 3D: Cycling of CO2

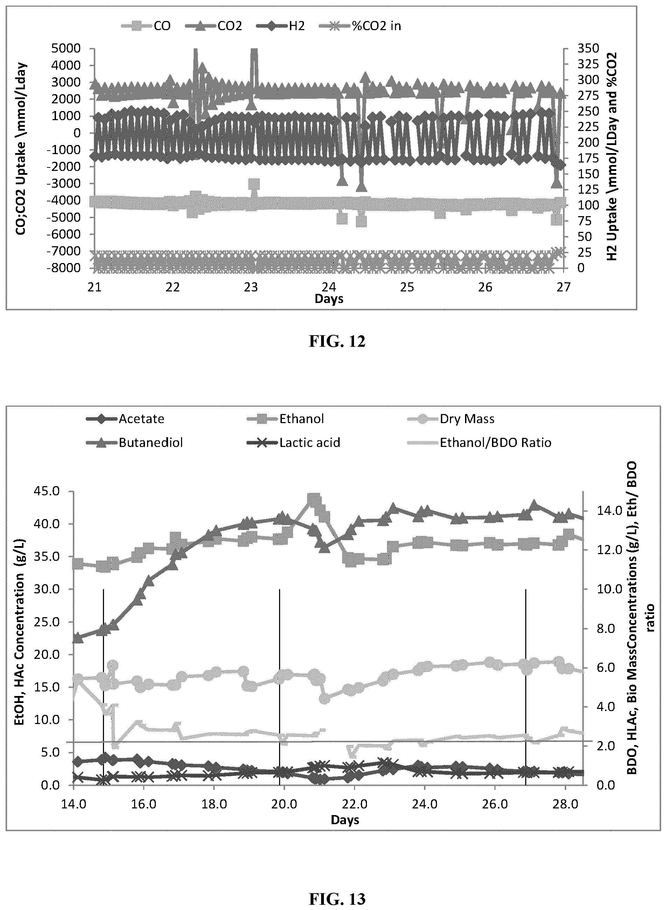

This experiment was performed to determine the effect of cycling the CO2 inlet concentration. The fermentation was set up so that the amount of CO2 in the inlet stream was cycled between 0% and 20% every hour. Figure 11 shows the metabolite production over the course of the experiment. The cycling of CO2 inlet concentrations had the effect of maintaining 2,3-BDO production at a slightly increased concentration. Figure 12 shows the uptake of various components of the inlet gas by the microbial culture over the duration of the experiment.

### 3E: Increase in CO2 concentration to the second reactor of a two reactor system.

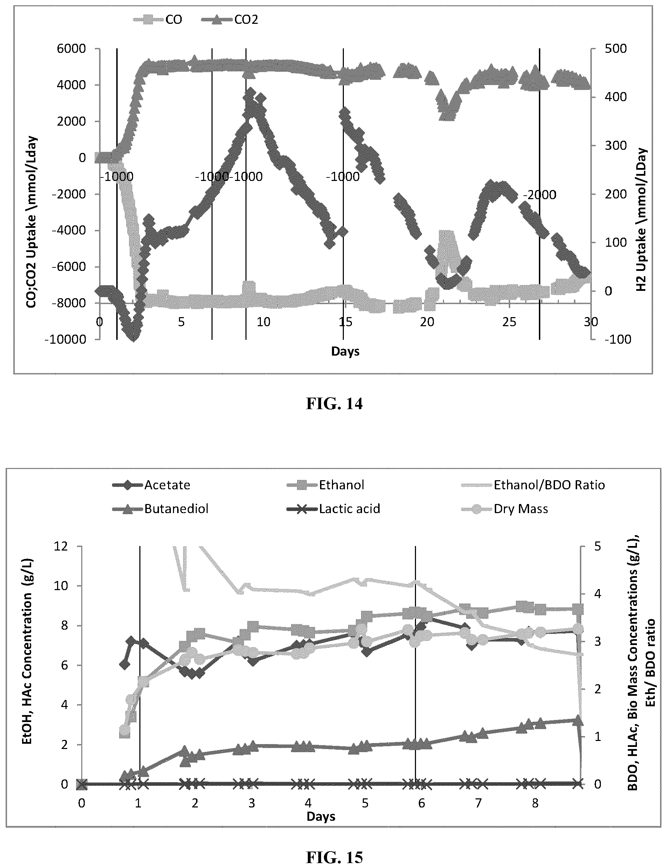

This experiment was designed to demonstrate the effect of passing the exit gas stream from a first bioreactor to the inlet stream of a second bioreactor thereby increasing the CO₂ concentration. Figure 13 shows plots of the metabolite concentration in the second bioreactor of the two reactor system between days 14 and 20 of the fermentation process. At day 14 the amount of CO2 provided to the second bioreactor from the first bioreactor was increased such that the total amount of CO2 in the second bioreactor went from 17.8% to 43.8%. Between days 14 and 21, the concentration of 2,3-BDO in the reactor increased from about 8g/L to about 14g/L. The ethanol to 2,3-BDO ratio decreased from 4:1 on day 14 to 2:1 around day 20 and remained relatively constant for the remainder of the experiment. Figure 14 shows the uptake of CO, CO₂ and H₂ for the microbial culture over the course of the fermentation.

### Example 4: Increasing 2,3-BDO production by controlling CO utilisation.

### Demonstrating effect of gas flow and agitation on metabolite production.

This experiment was designed to demonstrate the effect of changes in mass transfer on the metabolite production of a microbial culture. Over the course of the experiment, the agitation rates and gas flow were varied resulting in changes to the gas exiting the reactor, and the metabolite profile of the fermentation.

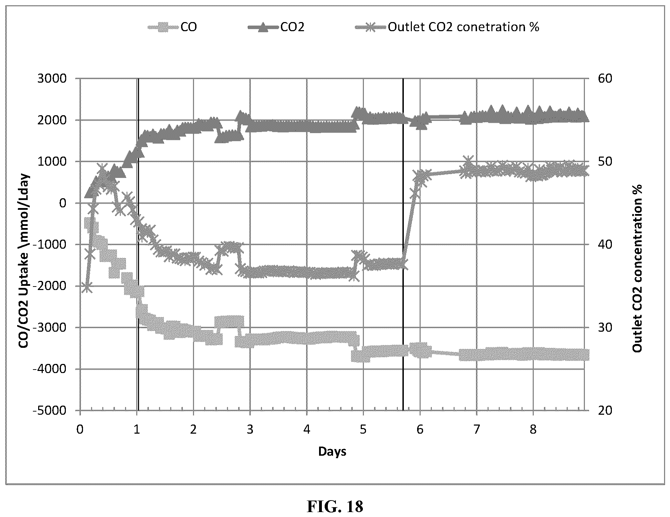

Referring to Figure 15, an increase in 2,3-BDO concentration can be seen from day 6 to day 8, corresponding to an increase in the agitation rate within the bioreactor and a decrease in the gas flow to the reactor. As shown in Figure 18, on day 5.6 CO uptake was kept constant but the utilisation of CO improved, hence CO2 in the outlet gas increased. This was done by increasing the agitation rate (rpm), and decreasing the gas flow so that the CO in the outlet gas decreased from 26 % to 12.5 %. The CO uptake stayed the same as the gas flow was reduced from 240ml/min/L to 160 ml/min/L. CO2 in the outlet stream increased from 37 % to 48 %. The CO utilisation increased from 53 % to 79 %. As a result of this increase the dissolved CO2 increased without any increase in CO uptake. The increase in CO utilisation positively correlated with an increase in 2,3-BDO production, as higher CO utilisation corresponds with more dissolved CO2.

### Effect of dissolved CO2 in the fermentation broth on the 2, 3-butanediol productivity rate.

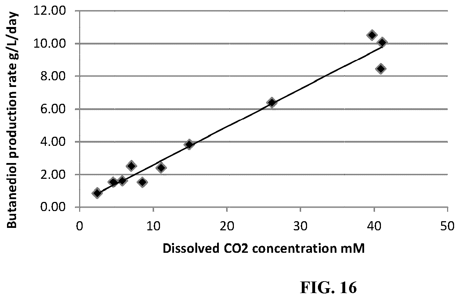

Combined data from a number of runs with different outlet CO₂ concentrations at different headspace pressure were plotted to show the relationship of dissolved CO₂ in the fermentation broth to the production of 2,3-butanediol. Figure 16 is a plot of dissolved CO₂ versus 2,3-BDO production rate. The plot shows that an increase in the amount of dissolved CO2 in the fermentation broth corresponds to an increase in the productivity rate of 2,3-butanediol.

The Table presents results from a number of experiments which again shows the correlation between dissolved CO₂ and 2,3-BDO concentration and productivity.

**The Table**

| **Run #** | **Calc. dissolved CO2 mM*** | **CO2 out %** | **BDO g/L** | **Inlet CO2 %** | **CO uptake mM/L/day** | **Data points** | **Normalised BDO g/L (4 mol CO uptake)** | **Production rate g/L/day** | **Normalised production rate (4 mol CO uptake)** |
|---|---|---|---|---|---|---|---|---|---|
| **1** | 2.39 | 10.62 | 0.5 | 0 | -4280 | Average ~ 3 days | 0.47 | 0.92 | 0.86 |
| **2** | 4.58 | 20.3 | 0.88 | 10 | -4201 | Average ~ 3 days | 0.84 | 1.6 | 1.52 |
| **3** | 5.76 | 25.5 | 0.96 | 15 | -4234 | Average ~ 3 days | 0.91 | 1.71 | 1.62 |
| **4** | 7 | 31.1 | 1.37 | 20 | -4071 | Average ~ 3 days | 1.35 | 2.58 | 2.54 |
| **5** | 8.5 | 37 | 0.86 | 18.5 | -3597 | Average ~ 3 days | 0.96 | 1.36 | 1.51 |
| **6** | 11.05 | 49 | 1.38 | 18.8 | -3595 | Average ~ 3 days | 1.54 | 2.18 | 2.43 |
| **7** | 14.89 | 66 | 2.22 | 50 | -4055 | Before crash | 2.19 | 3.89 | 3.84 |
| **8** | 26.1 | 50 | 5.3 | 19.2 | -5000 | Average day 9 - 12 | 4.24 | 8 | 6.40 |
| **9** | 39.7 | 498 | 10.38 | 20.5 | -5051 | Average day 4.3 - 6.0 | 8.22 | 13.3 | 10.53 |
| **10** | 40.9 | 48 | 6.48 | 18.5 | -6500 | Average day 7-8 | 3.99 | 13.75 | 8.46 |
| **11** | 41.1 | 46 | 10.38 | 22.3 | -5319 | Average day 6.3 - 9.3 | 7.81 | 13.4 | 1008 |

The invention has been described herein with reference to certain preferred embodiments, in order to enable the reader to practice the invention without undue experimentation. Those skilled in the art will appreciate that the invention is susceptible to invention includes all such variations and modifications. Furthermore, titles, headings, or the like are provided to enhance the reader's comprehension of this document, and should not be read as limiting the scope of the present invention.

The entire disclosures of all applications, patents and publications, cited above and below, if any, are hereby incorporated by reference.

The reference to any prior art in this specification is not, and should not be taken as, an acknowledgment or any form of suggestion that that prior art forms part of the common general knowledge in the United States of America or any country in the world

Throughout this specification and any claims which follow, unless the context requires otherwise, the words "comprise", "comprising" and the like, are to be construed in an inclusive sense as opposed to an exclusive sense, that is to say, in the sense of "including, but not limited to".

Further aspects of the invention are set out below:
1. A method for controlling the metabolic profile of a fermentation culture comprising at least one carboxydotrophic acetogenic microorganism, the method comprising:
   a. flowing a gaseous substrate comprising CO and CO₂ to a bioreactor comprising a culture of the microorganism in a liquid nutrient medium to produce at least one product derived from acetyl CoA and at least one product derived from pyruvate; and
   b. adjusting the amount of CO₂ dissolved in the liquid nutrient medium such that the production of at least one product derived from pyruvate is controlled.
2. The method of aspect 1, wherein the amount of CO₂ dissolved in the medium is increased such that the production of at least one product derived from pyruvate is increased.
3. The method of aspect 1, wherein the amount of CO₂ dissolved in the medium is decreased such that the production of at least on product derived from pyruvate is decreased.
4. The method of aspect 1, wherein the amount of CO₂ dissolved in the medium is adjusted by controlling the flow of CO₂ to the bioreactor.
5. The method of aspect 1, wherein the CO₂ partial pressure is adjusted in order to adjust the amount of CO2 dissolved in the liquid nutrient medium.
6. The method of aspect 1, wherein the fermentation is carried out at a pressure of greater than 250 kPag such that the concentration of CO₂ dissolved in the liquid nutrient medium is increased.
7. The method of aspect 1, wherein the fermentation is carried out at a pressure of less than 200 kPag such that the concentration of CO₂ dissolved in the liquid nutrient medium is reduced.
8. The method of aspect 1, wherein the concentration of CO₂ in the gaseous substrate provided to the bioreactor is from about 15% to about 65%.
9. The method of aspect 1, wherein the concentration of CO₂ in the gaseous substrate provided to the bioreactor is gradually increased over time.
10. The method of aspect 1, wherein the at least one product derived from pyruvate is selected from the group consisting of 2,3-butanediol, lactate, succinate, methyl ethyl ketone (MEK), 2-butanol, propanediol, 2-propanol, isopropanol, acetoin, isobutanol, citramalate, butadiene and poly lactic acid (PLA).
11. The method of aspect 1, further comprising monitoring the CO₂ concentration in an exit stream exiting the bioreactor in order to monitor the amount of CO₂ utilised by the culture within the bioreactor.
12. The method of aspect 1, wherein adjusting the amount of CO₂ dissolved in the liquid nutrient medium controls the ratio of pyruvate derived products to acetyl CoA derived products.
13. A method for producing one or more products by microbial fermentation of a gaseous substrate, the method comprising:
   a. receiving a gaseous substrate comprising CO in a first bioreactor comprising a culture of at least one carboxydotrophic acetogenic microorganism in a liquid nutrient medium;
   b. fermenting the gaseous substrate comprising CO to produce one or more liquid products and an exit gas comprising CO₂;
   c. passing the exit gas comprising CO₂ either back to the first bioreactor or to a second bioreactor, wherein said second bioreactor comprises a culture of at least one carboxydotrophic acetogenic microorganism in a liquid nutrient medium; and
   d. fermenting the exit gas comprising CO₂ in the first or second bioreactor to produce at least one product.
14. The method of aspect 13, wherein the exit gas comprising CO₂ is blended with at least one gaseous substrate prior to being fed to the second bioreactor.
15. The method of aspect 14, wherein at least one additional gaseous substrate is fed to the second bioreactor for use as a substrate in the fermentation.
16. The method of aspect 13, wherein the first bioreactor produces acetyl CoA derived products and pyruvate derived products and the ratio of acetyl CoA derived products to pyruvate derived products is from about 30:1 to about 20:1.
17. The method of aspect 13, wherein the second bioreactor produces a lower ratio of acetyl CoA derived products to pyruvate derived products than the first bioreactor.
18. The method of aspect 13, wherein the at least one product derived from pyruvate is selected from the group comprising 2,3-butanediol, lactate, succinate, methyl ethyl ketone (MEK), 2-butanol, propanediol, 2-propanol, isopropanol, acetoin, isobutanol, citramalate, butadiene and poly lactic acid (PLA).
19. The method of aspect 13, further comprising taking a exit gas stream produced from the second bioreactor and passing it to the first bioreactor for use as a substrate.
20. A method for controlling the metabolic profile of a fermentation culture comprising at least one carboxydotrophic acetogenic microorganism, the method comprising;
   a. flowing a gaseous substrate comprising CO to a bioreactor comprising a culture of the microorganism in a liquid nutrient medium to provide a fermentation broth; and
   b. increasing a rate of CO oxidation via a ferredoxin dependent carbon monoxide dehydrogenase to increase a level of reduced ferredoxin in the fermentation broth;
      wherein the increased level of reduced ferredoxin increases a rate of pyruvate fermentation from acetyl CoA.
21. The method according to any one of aspects 1, 13 or 20, wherein the at least one carboxydotrophic acetogenic microorganism is selected from the group consisting of *Moorella, Clostridium, Ruminococcus, Acetobacterium, Eubacterium, Butyribacterium, Oxobacter, Methanosarcina,* and *Desulfotomaculum.*
22. The method according to any one of aspects 1, 13 or 20, wherein the at least one microorganism is selected from the group consisting of *Clostridium autoethanogenum, Clostridium Ijundgahlii, Clostridium ragsdalei, Clostridium carboxydivorans* and *Clostridium coskatii.*

## Claims

1. A method for producing one or more products by microbial fermentation of a gaseous substrate, the method comprising:
e. receiving a gaseous substrate comprising CO in a first bioreactor comprising a culture of at least one carboxydotrophic acetogenic microorganism in a liquid nutrient medium;
f. fermenting the gaseous substrate comprising CO to produce one or more liquid products and an exit gas comprising CO₂;
g. passing the exit gas comprising CO₂ either back to the first bioreactor or to a second bioreactor, wherein said second bioreactor comprises a culture of at least one carboxydotrophic acetogenic microorganism in a liquid nutrient medium; and
h. fermenting the exit gas comprising CO₂ in the first or second bioreactor to produce at least one product.

2. The method of claim 1, wherein the exit gas comprising CO₂ is blended with at least one gaseous substrate prior to being fed to the second bioreactor.

3. The method of claim 2, wherein at least one additional gaseous substrate is fed to the second bioreactor for use as a substrate in the fermentation.

4. The method of claim 1, wherein the first bioreactor produces acetyl CoA derived products and pyruvate derived products and the ratio of acetyl CoA derived products to pyruvate derived products is from about 30:1 to about 20:1.

5. The method of claim 1, wherein the second bioreactor produces a lower ratio of acetyl CoA derived products to pyruvate derived products than the first bioreactor.

6. The method of claim 1, wherein the at least one product derived from pyruvate is selected from the group comprising 2,3-butanediol, lactate, succinate, methyl ethyl ketone (MEK), 2-butanol, propanediol, 2-propanol, isopropanol, acetoin, isobutanol, citramalate, butadiene and poly lactic acid (PLA).

7. The method of claim 1, further comprising taking a exit gas stream produced from the second bioreactor and passing it to the first bioreactor for use as a substrate.

8. A method for controlling the metabolic profile of a fermentation culture comprising at least one carboxydotrophic acetogenic microorganism, the method comprising;
c. flowing a gaseous substrate comprising CO to a bioreactor comprising a culture of the microorganism in a liquid nutrient medium to provide a fermentation broth; and
d. increasing a rate of CO oxidation via a ferredoxin dependent carbon monoxide dehydrogenase to increase a level of reduced ferredoxin in the fermentation broth;
wherein the increased level of reduced ferredoxin increases a rate of pyruvate fermentation from acetyl CoA.

9. The method according to claim 1 or 8, wherein the at least one carboxydotrophic acetogenic microorganism is selected from the group consisting of *Moorella, Clostridium, Ruminococcus, Acetobacterium, Eubacterium, Butyribacterium, Oxobacter, Methanosarcina,* and *Desulfotomaculum.*

10. The method according to claim 1 or 8, wherein the at least one microorganism is selected from the group consisting of *Clostridium autoethanogenum, Clostridium Ijundgahlii, Clostridium ragsdalei, Clostridium carboxydivorans* and *Clostridium coskatii.*
